(19) Europäisches Patentamt
European Patent Office
Office européen des brevets

(11) **EP 4 365 618 B1**

(12) **EUROPEAN PATENT SPECIFICATION**

(45) Date of publication and mention
of the grant of the patent:
**20.05.2026 Bulletin 2026/21**

(21) Application number: **23207754.5**

(22) Date of filing: **03.11.2023**

(51) International Patent Classification (IPC):
*G01R 33/56* (2006.01)    *G06N 3/045* (2023.01)
*G16H 30/40* (2018.01)    *G16H 50/20* (2018.01)

(52) Cooperative Patent Classification (CPC):
**G01R 33/5608; G06N 3/045; G16H 30/40;
G16H 50/20**

(54) **MULTILAYER PERCEPTRON FOR MACHINE-LEARNING IMAGE RECONSTRUCTION**

MEHRSCHICHTIGES PERZEPTRON ZUR MACHINE-LEARNING-BILDREKONSTRUKTION

PERCEPTRON MULTICOUCHE POUR LA RECONSTRUCTION D'IMAGE PAR APPRENTISSAGE AUTOMATIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.11.2022 US 202263422594 P**

(43) Date of publication of application:
**08.05.2024 Bulletin 2024/19**

(73) Proprietors:
• **University of Iowa Research Foundation**
**Iowa City, IA 52242 (US)**
• **Canon Medical Systems Corporation**
**Tochigi 324-0036 (JP)**

(72) Inventors:
• **JACOB, Mathews**
**Iowa City, 52242 (US)**
• **PRAMANIK, Aniket**
**Iowa City, 52242 (US)**
• **SHARMA, Samir Dev**
**Otawara-shi, 324-0036 (JP)**

(74) Representative: **Marks & Clerk LLP**
**15 Fetter Lane**
**London EC4A 1BW (GB)**

(56) References cited:
• **ZHANG HUIXIAN ET AL: "Multi-Contrast MRI Image Synthesis Using Switchable Cycle-Consistent Generative Adversarial Networks", DIAGNOSTICS, vol. 12, no. 4, 20 March 2022 (2022-03-20), CH, pages 816, XP093142108, ISSN: 2075-4418, DOI: 10.3390/diagnostics12040816**
• **YANG SERIN ET AL: "Continuous Conversion of CT Kernel Using Switchable CycleGAN With AdaIN", IEEE TRANSACTIONS ON MEDICAL IMAGING, IEEE, USA, vol. 40, no. 11, 5 May 2021 (2021-05-05), pages 3015 - 3029, XP011885204, ISSN: 0278-0062, [retrieved on 20211026], DOI: 10.1109/TMI.2021.3077615**
• **HEMANT KUMAR AGGARWAL ET AL: "MoDL: Model Based Deep Learning Architecture for Inverse Problems", ARXIV.ORG, CORNELL UNIVERSITY LIBRARY, 201 OLIN LIBRARY CORNELL UNIVERSITY ITHACA, NY 14853, 7 December 2017 (2017-12-07), XP080845783**
• **ANIKET PRAMANIK ET AL: "Adapting model-based deep learning to multiple acquisition conditions: Ada-MoDL", ARXIV.ORG, CORNELL UNIVERSITY LIBRARY, 201 OLIN LIBRARY CORNELL UNIVERSITY ITHACA, NY 14853, 21 April 2023 (2023-04-21), XP091491369**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Processed by Luminess, 75001 PARIS (FR)

**Description**

CROSS-REFERENCE TO RELATED APPLICATIONS

The present application claims the benefit of priority to provisional Application No. 63/422,594, filed November 4, 2022.

BACKGROUND

FIELD

The present disclosure relates a method for image reconstruction or filtering using a machine-learning network system including a multilayer perceptron model.

DESCRIPTION OF THE RELATED ART

Magnetic Resonance Imaging (MRI) provides an excellent soft tissue contrast at the expense of long acquisition times. Several acceleration methods including parallel MRI (PMRI), compressed sensing, and low-rank were introduced to speed up the acquisition. The corresponding reconstruction algorithms often pose the image recovery from under-sampled measurements as a regularized optimization scheme. In the recent years, deep-learning (DL) based algorithms have shown immense power in learning an approximate distribution/manifold of images, giving improved reconstruction performance over non-DL methods. These methods include direct inversion schemes and model-based strategies. Direct inversion schemes use a CNN (e.g., UNET, ResNet, etc.) to map/invert an under-sampled image to a fully sampled one. The mapping does not incorporate knowledge about data acquisition physics. Model-based deep-learning algorithms ("MoDL") integrate a CNN-based learned model with a physical model encoding the data acquisition process, to obtain a solution consistent with the measurements obtained. These algorithms are trained using algorithm unrolling in which an iterative algorithm is unrolled assuming a finite number of iterations, followed by end-to-end training. Such schemes have shown benefit in leveraging data acquisition models through improved performance over direct inversion schemes. These algorithms often require fewer training datasets compared to direct inversion schemes, and also have the ability to incorporate multiple blocks of priors for regularizing the solution. Finally, these model-based algorithms offer improved performance because the learned CNN representation is closely linked with the measurement scheme.

A challenge with the model-based schemes, which restrict their clinical deployment, however, is the dependence of the learned representation on the specific measurement scheme. It is well known that changes in the measurement model from the one the network is trained for can result in degradation in performance. While the training procedure can be modified to include multiple measurement operators to reduce the sensitivity of the learned representation to the measurement scheme, this approach often comes at the expense of reduced performance. Further, a typical MRI protocol often consists of several different sequences, each with different acquisition settings that differ in signal-to-noise ratio, acceleration, and matrix sizes. These parameters can also change depending on the field strength at which the data is acquired, and the image content will also significantly differ depending on the anatomy.

In order to provide optimum reconstruction performance, unrolled models need to be trained separately for each kind of acquisition. The regularization parameter also needs to be adapted based on the signal-to-noise ratio and under-sampling rate. Although an optimized model would provide the best possible performance, there are many possible combinations of parameters, and training a model for each of them would be challenging due to the lack of availability of data. In addition, storing multiple networks would require lots of memory, and switching between the models depending on subtle changes in the acquisition settings can be inconvenient.

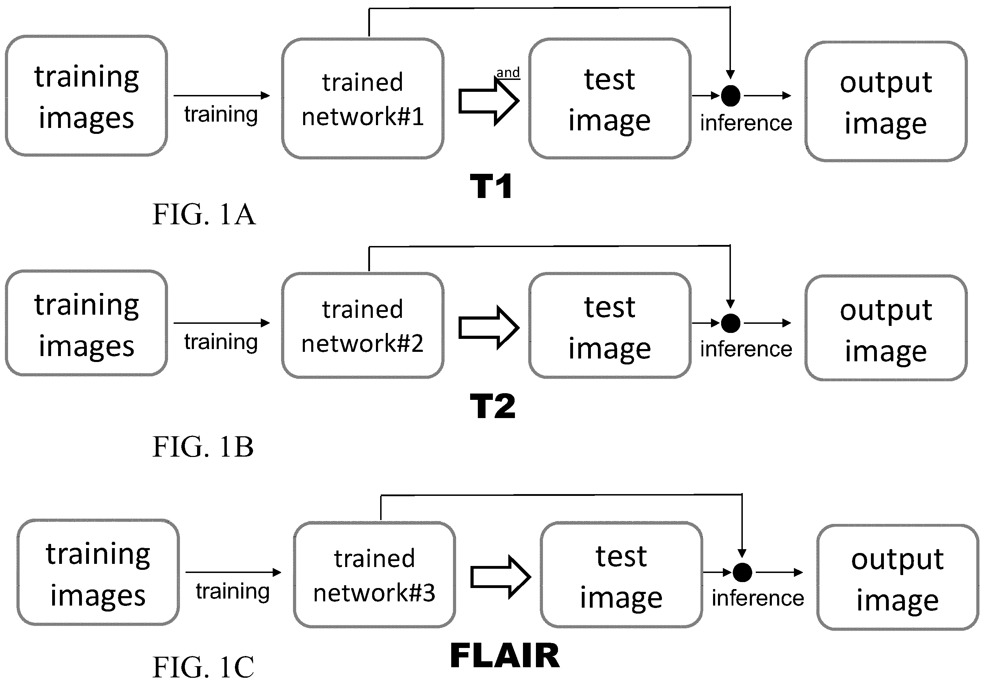

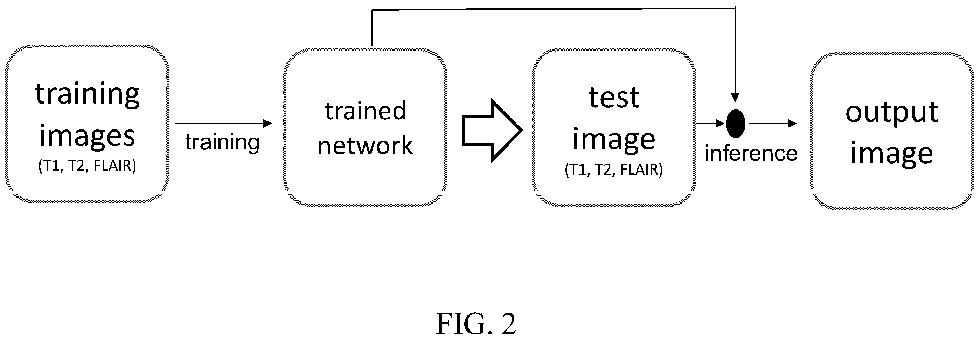

Magnetic Resonance Imaging (MRI) is a diverse imaging modality and is capable of imaging many different anatomies, contrasts, planes, etc. at different field strengths. Machine learning (ML) reconstruction is often trained for all of the different possible applications, e.g., different anatomies, contrasts, planes, and field strengths, to achieve good performance. As shown in FIGs. 1A-1C, one option (MoDL-I) is to train a neural network on each individual scanning application, e.g., 1.5T axial FLAIR brain, 1.5T axial T2 brain, 3T sagittal T1 brain, etc. Alternatively, another option (MoDL) is to train a single universal network to be used for all applications, as shown in FIG. 2. However, both approaches have limitations. For example, training one network per application can result is a complex set of networks that may be too complex to deploy. Further, large datasets are required to train a network for each different application. Moreover, training one universal network might degrade the overall performance as the network must learn one parameter set that is suitable for all possible applications.

Zhang Huixian ET AL: "Multi-Contrast MRI Image Synthesis Using Switchable Cycle-Consistent Generative Adversarial Networks", Diagnostics, vol. 12, no. 4, 20 March 2022 (2022-03-20), page 816 relates to a CycleGAN model for image synthesis between multi-contrast brain MRI images.

## EP 4 365 618 B1

YANG SERIN ET AL: "Continuous Conversion of CT Kernel Using Switchable CycleGAN With AdaIN", IEEE TRANSACTIONS ON MEDICAL IMAGING, IEEE, USA, vol. 40, no. 11, 5 May 2021 (2021-05-05), pages 3015-3029 relates to a continuous unsupervised kernel conversion method using cycle-consistent generative adversarial network with adaptive instance normalization.

HEMANT KUMAR AGGARWAL ET AL: "MoDL: Model Based Deep Learning Architecture for Inverse Problems", ARXIV.ORG, CORNELL UNIVERSITY LIBRARY, 201 OLIN LIBRARY CORNELL UNIVERSITY ITHACA, NY 14853, 7 December 2017 (2017-12-07) relates to a model based image reconstruction framework with a convolution neural network based regularization prior.

## SUMMARY

In a first aspect, an apparatus configured to reconstruct or filter medical image data as recited in claim 1 is provided.

In a second aspect, a method for reconstructing or filtering medical image data as recited in claim 7 is provided.

In a third aspect, a computer-readable medium as recited in claim 12 is provided.

## BRIEF DESCRIPTION OF THE DRAWINGS

The application will be better understood in light of the description which is given in a non-limiting manner, accompanied by the attached drawings in which:

FIGs. 1A, 1B, and 1C illustrate three approaches to training machine-learning networks to perform MRI reconstruction for correspondingly different applications;

FIG. 2 illustrates a universal approaches to training a single machine-learning network to perform MRI reconstruction for different applications;

FIG. 3 is a flowchart of a method for training and using a machine-learning network system according to embodiments of the present disclosure;

FIG. 4 is a flowchart of a method for training and using a machine-learning network system according to one embodiment of the present disclosure;

FIG. 5 is a flowchart of a method for using a machine-learning network system according to one embodiment of the present disclosure

FIG. 6A is a comparison of results of different methods according to one embodiment of the present disclosure for a 1.5T FLAIR example;

FIG. 6B is a comparison of results of different methods according to one embodiment of the present disclosure for a 1.5T T2 example;

FIG. 6C is a comparison of results of different methods according to one embodiment of the present disclosure for a 3T T1 example;

FIG. 6D is a comparison of results of different methods according to one embodiment of the present disclosure for another 3T T1 example;

FIG. 7 is a summary of a comparison of results of different methods according to one embodiment of the present disclosure for different examples;

FIG. 8 is an illustration of a medical imaging system configured to implement a method of improved spectrometer processing, according to an exemplary embodiment of the present disclosure; and

FIG. 9 is a schematic block diagram of a magnetic resonance imaging system, according to an exemplary implementation of the present disclosure.

## DETAILED DESCRIPTION

To overcome the above challenges, the present disclosure uses a conditional unrolled architecture, termed Meta-MoDL. In one embodiment, the method alternates between data-consistency and CNN denoising blocks. In the present disclosure, the output of each CNN layer is modulated by a set of scalar weights, which are dependent on conditional vectors representing the acquisition setting. Similarly, the regularization parameter $\lambda$ that balances the data-consistency and denoising penalties in a model-based scheme is also made dependent on the conditional vectors. This modulation enables the selection of features generated by the CNN based on acquisition information. The dependence between the scalar weights and the conditional vectors is modeled by a multi-layer perceptron (MLP) model that maps a conditional vector to a vector of scaling factors and regularization parameter. The parameters learned by the MLP are shared across iterations.

The number of free parameters in the MLP is around 5% of the parameters in the CNN such that the disclosed adaptation involves minimal overhead. This architecture enables the adaptation of the network to different acquisition settings, which allows training of the network using data from different acquisition settings. In the present disclosure, differences in sequences, acceleration factors, and field strength are accounted for. As discussed below, the joint training strategy enables the combination of information from multiple acquisitions settings, and hence is more training-data efficient than the individual training of unrolled networks for each acquisition setting.

Consider recovery of an image $\mathbf{x} \in \mathcal{C}^{\mathcal{N}}$ from its set of measurements obtained through a parallel MRI acquisition satisfying,

$$\mathbf{b} = A(\mathbf{x}) + \mathbf{n} \qquad\qquad (1)$$

where A is a linear operator embedding point-wise multiplication with coil sensitivity maps C, Fourier transform operator F and a sub-sampling operator S. The vector $\mathbf{b}$ represents a set of noisy measurements while $\mathbf{n}$ denotes additive Gaussian noise.

Several regularized inversion strategies are introduced to make the recovery well-conditioned. For example, the recovery can be posed as an optimization problem:

$$\arg \min_{\mathbf{x}} \; (\lambda \,/\, 2) \, \|A(\mathbf{x}) \; - \; \mathbf{b}\|_2^2 + R(\mathbf{x}) \qquad\qquad (2)$$

where $R(\mathbf{x})$ is a regularization term constraining the solution by using prior information about $\mathbf{x}$. The scalar $\lambda$ is a tunable parameter balancing the effect of DC and prior term. Traditional regularizers include handcrafted priors such as $l_1$ norm of wavelets, total variation, low-rank, structured low-rank methods and sparsity in other transform domains.

Plug and play (PnP) models rely on off-the-shelf or pretrained CNN denoisers to offer improved reconstruction. These methods consider an iterative proximal gradients algorithm used for sparse recovery; the proximal mapping steps in these algorithms are often replaced by denoisers to obtain improved results than regularized inversion using classical penalties. The above regularization schemes including PnP models require the tuning of the regularization parameter $\lambda$ to obtain good reconstructions.

Deep unrolled algorithms offer further improvement in performance. Unrolled algorithms assume a specific **A** operator and unroll the above iterative proximal gradients algorithm, assuming a finite number of iterations. The resulting deep network which alternates between data consistency blocks and deep learning blocks is trained in an end-to-end fashion, such that the reconstructed image matches the original image. The regularization parameter $\lambda$ is also optimized during training. Deep unrolled methods train the CNN blocks such that the reconstructed image best matches the fully sampled image.

Empirical results show that the unrolled optimization offers improved performance than model-agnostic PnP methods because it learns a representation that is ideal for a specific measurement operator. However, the performance improvement often comes at the expense of reduced generalizability to measurement conditions. In particular, the unrolled model trained for a specific **A** may result in sub-optimal results for another measurement scheme (e.g., different under-sampling rate or measurement condition). The generalizability may be improved by training with multiple measurement models, at the expense of reduced performance.

MRI offers improved visualization of tissue using multiple contrasts (e.g $T$1, $T$2, FLAIR). Each of these measurement schemes use very different acquisition settings. For instance, the signal-to-noise ratio (SNR) of inversion-recovery based FLAIR acquisitions are often lower than that of $T$1 or $T$2 scans. Similarly, the SNR of images vary with scanner field strengths. In many cases, the specific sampling patterns and acceleration factors are chosen to match the demands of the scan.

When unrolled architectures are used, one has to train multiple CNN modules for each of the above acquisition setups to get optimal performance. The acquisition of large fully sampled exemplar datasets for each of the settings is often challenging. In addition, storage of models for each different contrasts, field strengths, and acceleration factors is also required. While one may train a single model for different acquisition settings, this approach often translates to degraded performance as discussed before.

The present disclosure overcomes these limitations by a conditional MoDL architecture, where a single model is adapted depending on the acquisition settings. Because the network parameters are expected to vary with image contrast, signal-to-noise ratio, and acquisition settings, the present disclosure uses these parameters as conditional vectors denoted by $m$; the conditional vectors can be derived from the meta-data of the acquisition.

Similar to the traditional MoDL, the proposed denoising module consists of multiple convolution layers. A difference with MoDL is that each feature is scaled by weights, which are derived from the conditional vector as $P(m)$. Here, P is a function that is realized by a multilayer perceptron (MLP). The modulation of the features allows this approach to emphasize or de-emphasize specific features, depending on the condition vectors. The regularization parameter $\lambda$ is expressed as a function of the conditional variables $m$. This adaptation of the regularization parameters allows the ML network to account for differences in signal-to-noise ratio in the datasets.

The Meta-MoDL formulation can be compactly represented as

$$\mathbf{x} = \arg\min_{\mathbf{x}}(\lambda(\mathrm{m})/2) \, \|\mathrm{A}(\mathbf{x}) - \mathbf{b}\|_2^2 + \|\mathrm{N}(\mathbf{x}, \theta, \mathrm{P}(m))\|_2^2 \tag{3}$$

where $M(m) = [P(m), \lambda(m)]$ is an MLP mapping the conditional vector $m$ to an appropriate vector $P(m)$ containing feature scaling factors for CNN N. Both CNN and MLP parameters are repeated across iterations. The network is unrolled for a fixed number of iterations, alternating between the data consistency (DC) enforcing step in Equation (5) below and the residual CNN denoiser in Equation (4) below.

The equations for alternating blocks are

$$\mathbf{z}_k = \mathrm{D}(\mathbf{x}_k, \theta, \mathrm{P}(m)) \tag{4}$$

$$\mathbf{x}_{k+1} = (\mathrm{A}^H\mathrm{A} + \lambda(m)\mathbf{I})^{-1}(\mathrm{A}^H\mathbf{b} + \lambda(m)\mathbf{z}_k), \tag{5}$$

$$k = 1, ..,$$

which provide conditional-vector dependent denoised output $\mathbf{z}$ and updated image $\mathbf{x}$. Here, $K$ is the number of unrolls or iterations. Note that the same denoiser block D is used for all the iterations $k = 1, ..,$.

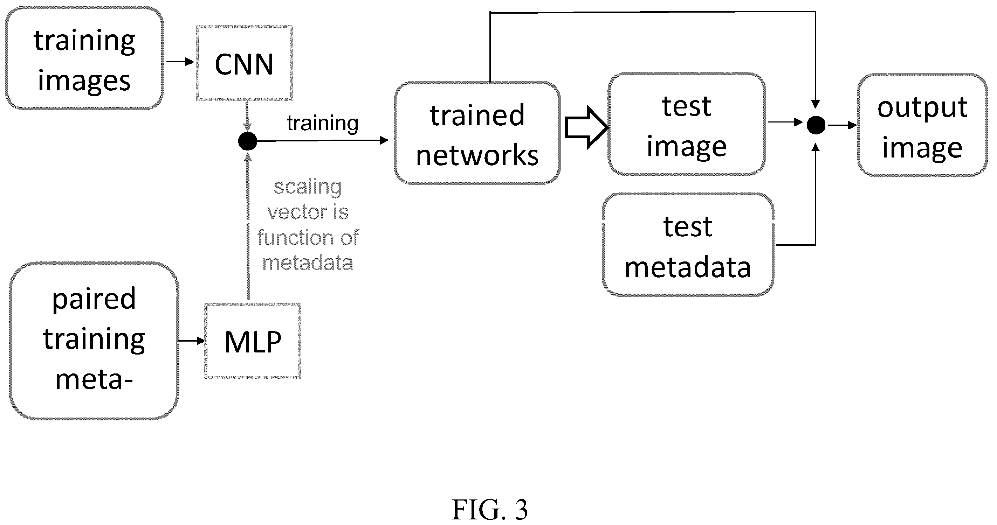

The Meta-MoDL framework with its CNN and MLP architectures is shown in FIGs. 3-5.

As shown in FIG. 3, in one embodiment, one ML network system is trained for all different applications using both the imaging data and imaging metadata (anatomy, contrast, etc.). In the CNN, each output feature of the intermediate network layers is scaled, wherein the scaling is a learned nonlinear mapping of the meta-parameters. As shown in FIG. 3, the nonlinear mapping is modelled using a multilayer perceptron (MLP), and is learned from all of the training datasets. The use of the MLP with a finite capacity constrains the scaling for different settings, facilitates the merging of information across different type of images and settings, and reduces training data demand as less data is needed per application.

The approach shown in FIG. 3 keeps the complexity of the network low, as it is not necessary to learn and deploy one network per application, as is needed for a set of individual networks. Further, this approach improves performance using one universal ML network system with the same amount of training data

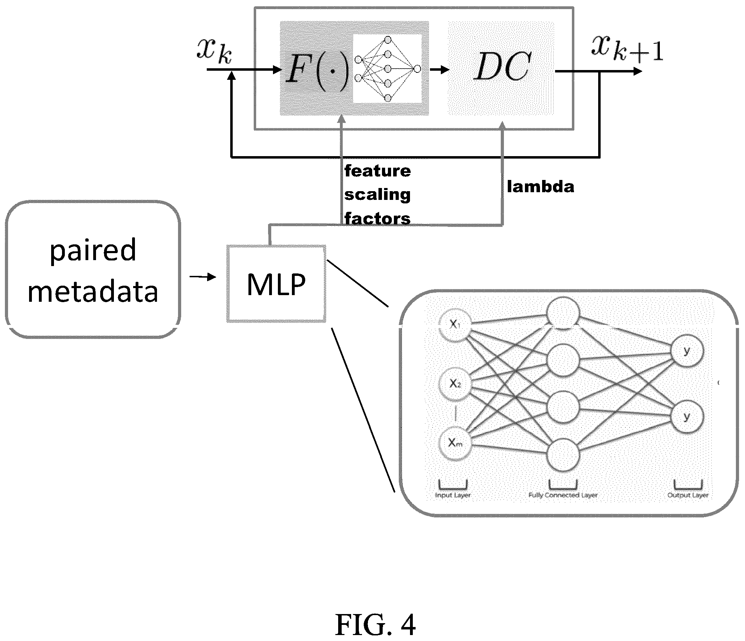

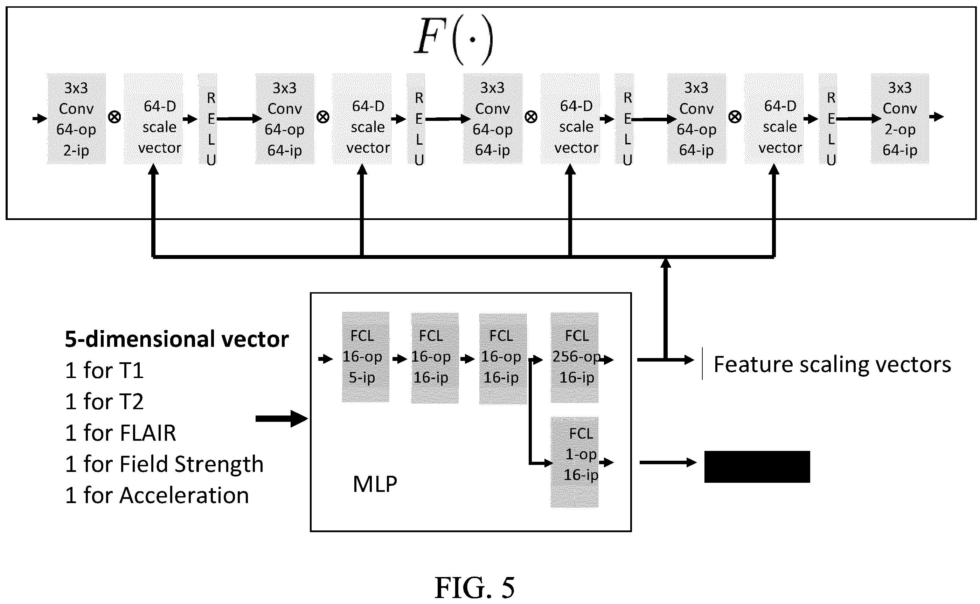

As shown in FIG. 4, the MLP is a fully connected network that receives metadata parameters of the corresponding image input to the CNN. The metadata parameters can include information related to anatomy, field strength, etc. for an MRI application. The outputs of the MLP can include the regularization parameter lambda ($\lambda$) and a set of feature scaling vectors used to scale the intermediate layers of the CNN, as shown in more detail in the example of FIG. 5.

In one embodiment, as shown in FIG. 5, the CNN F( ) consists of five 3 x 3 convolution layers with 64 output channels for each of them, except the last layer contains two. The 64 output features from the intermediate layers are scaled by corresponding 64-dimensional vector obtained from the MLP which is followed by ReLU non-linearity. In one embodiment, the MLP consists of five fully connected (1 x 1 convolution) layers with 16 output channels for the intermediate layers. In one embodiment, the output layer of the MLP provides a vector of size 256 and a scalar lambda ($\lambda$). In one embodiment, the metadata is a five-dimensional vector consisting of a bit each for T1, T2, FLAIR, field-strength, and acceleration.

As noted above, in one embodiment, a five-layer CNN is used in which each convolution layer consists of 3 x 3 filters with 64 channels per layer, except the last layer. Thus, the five-layer CNN has 113,000 parameters. In one embodiment, the MLP has four hidden layers, each with 64 features. Thus, the total number of learnable parameters in the MLP is 5517, which is around 5% of the parameters of the five-layer CNN. Thus, Meta-MoDL has approximately 5% additional trainable parameters compared to MoDL.

MRI images used in the embodiments of the present disclosure were obtained as follows, for example. Fully sampled brain MR raw-data with $T1$, $T2$ and FLAIR contrasts were collected from human subjects on an Orian 1.5T and a Galan 3T system using a 16-channel head/neck coil (Canon Medical Systems Corporation, Tochigi, Japan). We denote a dataset's type as a combination of contrast and field strength. For instance, $T1$ data collected from 3T scanner is denoted as $T1$-3T. The fully sampled data was acquired at six different settings: $T1$-3T, 2-3T, FLAIR-3T, $T1$-1.5T, $T2$-1.5T and FLAIR-1.5T, respectively. The 2D matrix size was set as 512 x 320 for all scans. The under-sampled raw-data have been generated retrospectively using masks with 1-D variable density under-sampling, along the phase encoding direction.

One example of a training procedure is as follows. The network was trained using five different types of datasets ($T2$-3T, FLAIR-3T, $T2$-1.5T, $T1$-1.5T, and FLAIR-1.5T). The $T1$-3T data was not included in the training to determine the ability of the network to extrapolate the findings to an unseen combination. During training, each of the above datasets were under-sampled at four different acceleration factors (1.8, 2.5, 3.5 and 4.0), resulting in a total of 20 different acquisition settings. To study the effect of dataset size, training was performed with S subjects per acquisition setting, with S varying from one to four. The training dataset consists of twenty training datasets, and the testing was performed on two datasets per acquisition setting (total of 10 datasets).

The acquisition settings were summarized by a five-dimensional conditional vector. The first three entries encode the type of the acquisition (T1, T2 or FLAIR) in a one-hot fashion. The fourth component is binary, indicating the field strength (3T or 1.5T). The last entry is a floating point number, which represents the acceleration factor.

The denoiser was pre-trained with a single unrolling step (K = 1) in Equations (4) and (5); the D trained with K = 1 initialization was used to train the network with K = 3. The same training strategy is used for both MoDL and MoDL-I. The models were optimized using mean-squared error (MSE) loss for 500 epochs with the Adam optimizer at a learning rate of $10^{-4}$.

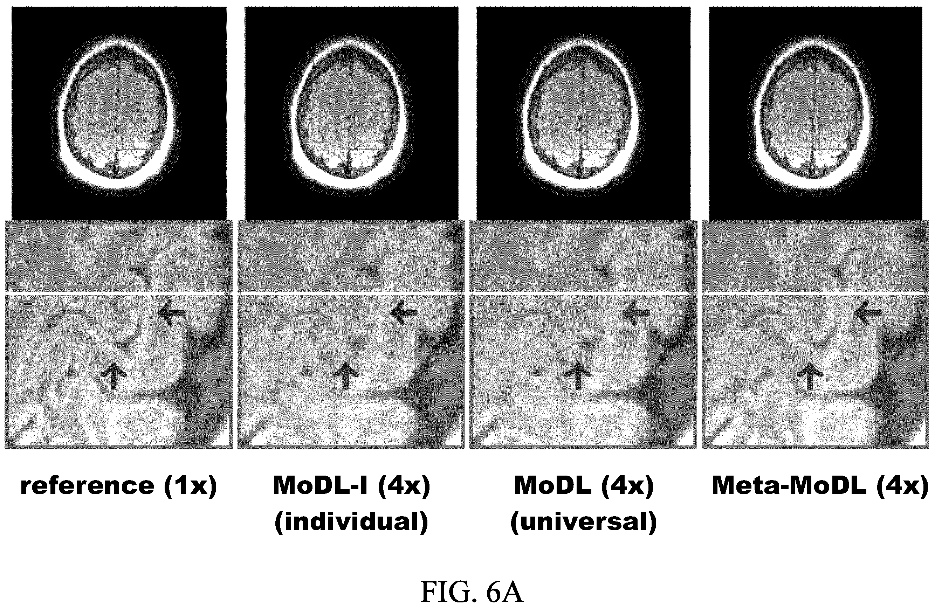

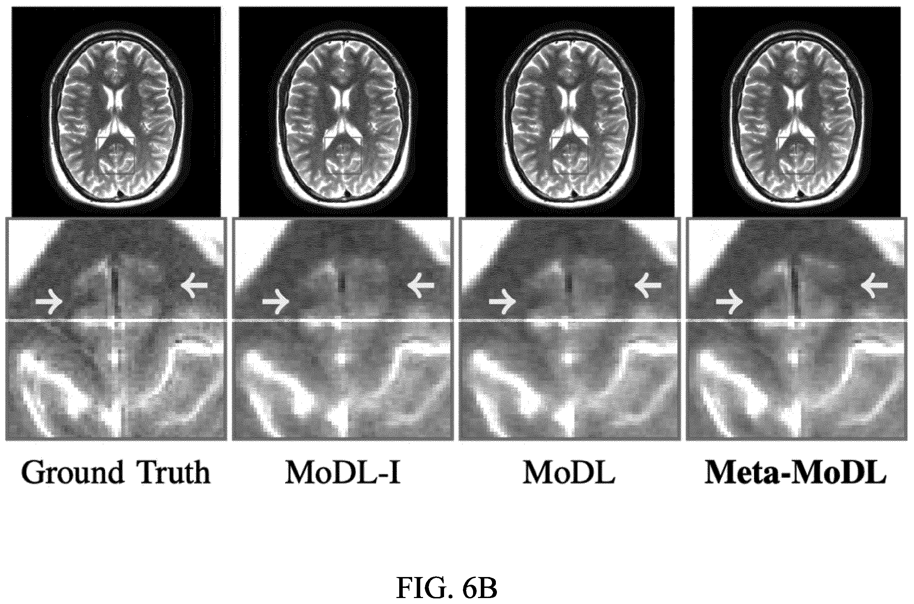

FIGs. 6A and 6B show the reconstructed images for FLAIR 1.5 T and T2 1.5T, respectively. For FLAIR-1.5T, the arrows indicate a better preservation of details in Meta-MoDL which seems lost in the other two. In T2-1.5T, the blue arrows indicate relatively sharper edges for Meta-MoDL. Meta-MoDL also seems to preserve details more accurately compared to MoDL and MoDL-I. In terms of SNR, Meta-MoDL offers an improved margin of 0.6-0.8 dB. Note that MoDL learns a single $\lambda$ for all the contrasts and hence suffers from lack of flexibility in balancing the effect of DC and prior term. MoDL-I requires several models since it requires a model to be trained for every acquisition setting including contrast, acceleration, field strength etc. which is prohibitive. Meta-MoDL addresses these limitations with a conditional framework as shown in these performance comparisons.

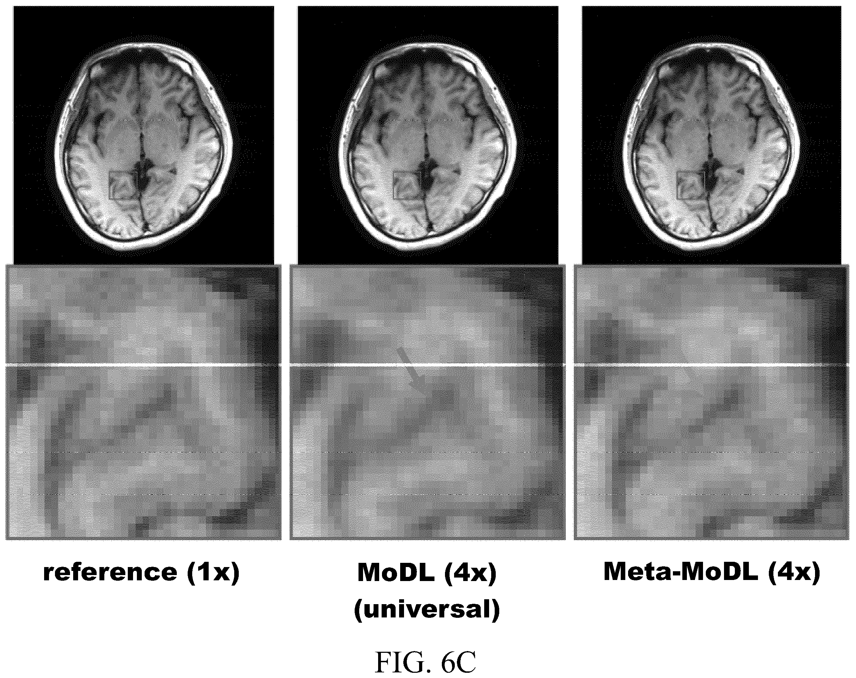

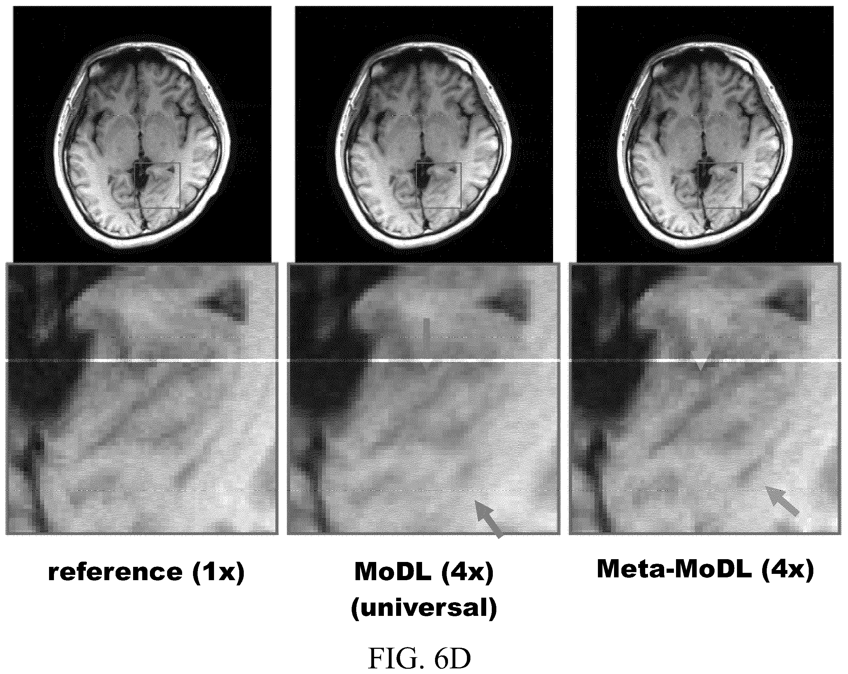

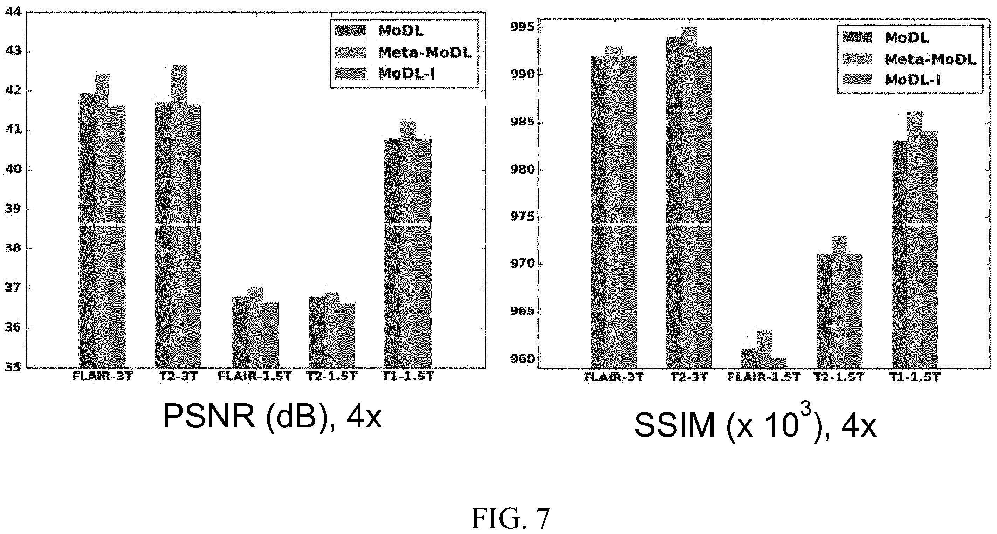

The experiments shown in FIGs. 6A and 6B correspond to acquisition settings that were used in training. FIGs. 6C and 6D show results for images or a 3T-T1 application for an inputs not seen during the training phase. FIG. 7 illustrates that Meta-MoDL results in better peak signal-to-noise ratio (PSNR) and structural similarity index (SSIM) for all applications compared to a universal network (MoDL) approach and an individual network (MoDL-I) approach.

Current clinical protocols often include multiple MRI scans with different acquisition parameters including contrasts, acceleration, matrix sizes, and resolution. Similarly, the data acquired from scanners with different field strengths differ significantly in signal-to-noise ratio. Unrolled models offer improved performance, when the deep network is trained for each specific acquisition setting. Unfortunately, the training of specific unrolled models for each acquisition setting is challenging because it is difficult to obtain sufficient training data for each specific acquisition setting. In addition, multiple trained models need to be stored and selected from at inference, depending on the specific acquisition setting. While one may train a single network for multiple acquisition settings, this can translate to decreased performance.

The Meta-MoDL framework overcomes the above challenges that restrict the clinical deployment of unrolled deep network architectures. The disclosed unrolled architecture includes a traditional CNN denoising network, whose feature weights are modulated by acquisition setting dependent weights. The regularization penalty is also modulated. The feature weights and the regularization penalty are derived from the acquisition parameters by an MLP, whose parameters are learned from the data. The number of parameters in the MLP is around 5% of that of the number of parameters of the unrolled network.

The experiments show that the modulation of the feature weights and regularization parameter allows the unrolled network to adapt to the specific acquisition setting, thereby offering improved performance than using a fixed network for all the acquisition conditions. In addition, the experiments show that the proposed approach of joint learning of the reconstructions for all the experiment settings is more data efficient than learning a separate unrolled network for each acquisition condition. The experiments also show that the MLP can extrapolate the parameters for acquisition settings that may not be included in the training datasets. The disclosed approach also makes it easy to deploy deep unrolled networks in a clinical setting. Rather than using multiple networks for each specific acquisition setting, the parameters can be adapted depending on the metadata.

In this disclosure, we only considered the dependence of the model parameters on acceleration, field strength, and three different contrasts. However, the framework can be expanded to a single universal network that can adapt to variations including anatomy, signal to noise ratio, different coil arrays, as well as differences in field of view and matrix sizes.

Unlike traditional unrolled DL methods, the disclosed embodiments of Meta-MoDL have learnable parameters which are functions of acquisition information of the dataset to be recovered. This approach provides a single network for image recovery from multiple acquisition settings including contrasts, field-strengths and acceleration factors. The ability of Meta-MoDL to adapt to the acquisition condition translates to improved performance over traditional unrolled methods. The joint training of the Meta-MoDL network on multiple datasets was seen to be more training-data efficient than training individual unrolled networks for each specific acquisition condition. The results also show that Meta-MoDL is able to extrapolate to acquisition settings, which are not seen during training. The lightweight architecture is thus expected to make the deployment of unrolled architecture to clinical settings more efficient.

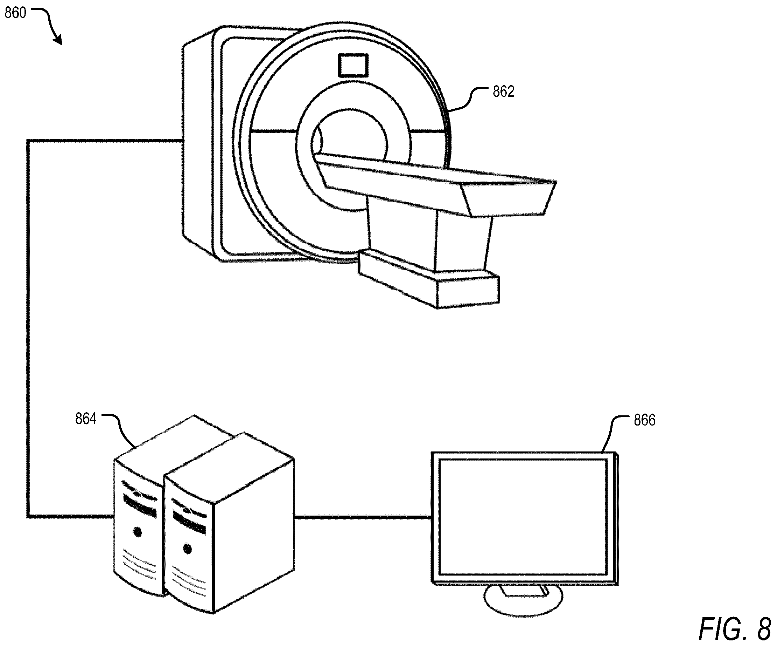

FIG. 8 illustrates an example embodiment of a medical-imaging system 860 within which embodiments of the present disclosure can be implemented. The medical-imaging system 860 includes at least one scanning device 862, one or more image-generation devices 864, each of which is a specially-configured computing device (e.g., a specially-configured desktop computer, a specially-configured laptop computer, a specially-configured server), and a display device 866.

The scanning device 862 is configured to acquire scan data by scanning a region (e.g., area, volume, slice) of an object (e.g., a patient). The scanning modality may be, for example, magnetic resonance imaging (MRI), computed tomography (CT), positron emission tomography (PET), X-ray radiography, and ultrasonography.

The one or more image-generation devices 864 obtain scan data from the scanning device 862 and generate an image of the region of the object based on the scan data. To generate the image, for example during intermediate image generation or during final image reconstruction, the one or more image-generation devices 864 may perform a reconstruction process on the scan data. Examples of reconstruction processes include GRAPPA, CG-SENSE, SENSE, ARC, SPIRIT, and LORAKS, and compressed sensing.

In an embodiment, after the one or more image-generation devices 864 generate the image, the one or more image-generation devices 864 send the image to the display device 864, which displays the image.

In another embodiment, and further to the above, the one or more image-generation devices 864 may generate two images from the same scan data. The one or more image-generation devices 864 may use different reconstruction processes to generate the two images from the same scan data, and one image may have a lower resolution than the other image. Additionally, the one or more image-generation devices 864 may generate an image.

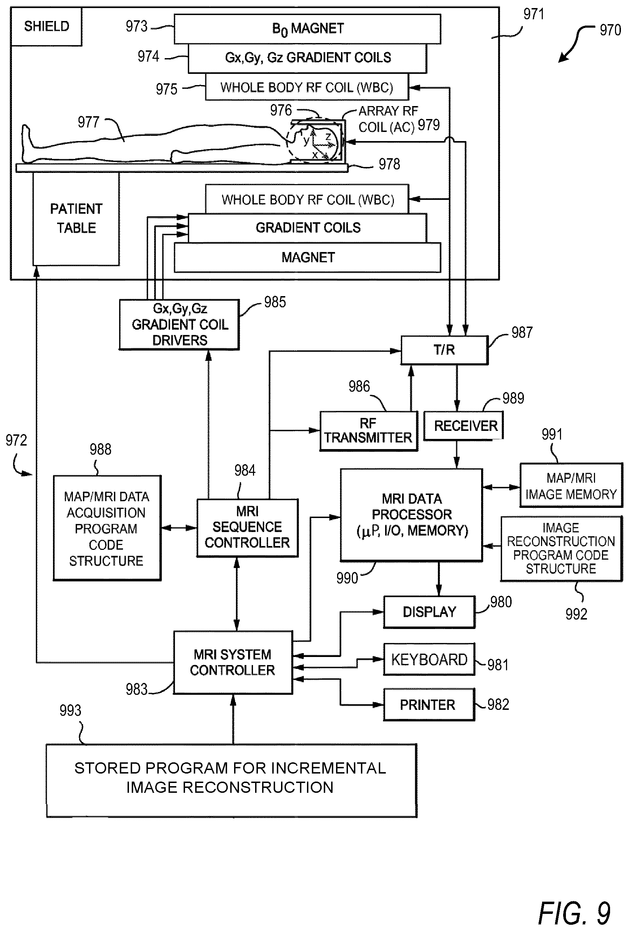

Referring now to FIG. 9, a non-limiting example of a magnetic resonance imaging (MRI) system 970 is shown. The MRI system 970 depicted in FIG. 9 includes a gantry 971 (shown in a schematic cross-section) and various related system components 972 interfaced therewith. At least the gantry 971 is typically located in a shielded room. The MRI system geometry depicted in FIG. 9 includes a substantially coaxial cylindrical arrangement of the static field $B_0$ magnet 973, a Gx, Gy, and Gz gradient coil set 974, and a large whole-body RF coil (WBC) assembly 975. Along a horizontal axis of this cylindrical array of elements is an imaging volume 976 shown as substantially encompassing the head of a patient 977 supported by a patient table 978.

One or more smaller array RF coils 979 can be more closely coupled to the patient's head (referred to herein, for example, as "scanned object" or "object") in imaging volume 976. As those in the art will appreciate, compared to the WBC (whole-body coil), relatively small coils and/or arrays, such as surface coils or the like, are often customized for particular body parts (e.g., arms, shoulders, elbows, wrists, knees, legs, chest, spine, etc.). Such smaller RF coils are referred to herein as array coils (AC) or phased-array coils (PAC). These can include at least one coil configured to transmit RF signals into the imaging volume, and a plurality of receiver coils configured to receive RF signals from an object, such as the patient's head, in the imaging volume 976.

The MRI system 970 includes a MRI system controller 983 that has input/output ports connected to a display 980, a keyboard 981, and a printer 982. As will be appreciated, the display 980 can be of the touch-screen variety so that it provides control inputs as well. A mouse or other I/O device(s) can also be provided.

The MRI system controller 983 interfaces with a MRI sequence controller 984, which, in turn, controls the Gx, Gy, and Gz gradient coil drivers 985, as well as the RF transmitter 986, and the transmit/receive switch 987 (if the same RF coil is used for both transmission and reception). The MRI sequence controller 984 includes suitable program code structure 988 for implementing MRI imaging (also known as nuclear magnetic resonance, or NMR, imaging) techniques including parallel imaging. Moreover, the MRI sequence controller 984 includes processing circuitry to execute the scan control process illustrated in Figure 1. The MRI sequence controller 984 can be configured for MR imaging with or without parallel imaging. Moreover, the MRI sequence controller 984 can facilitate one or more preparation scan (pre-scan) sequences, and a scan sequence to obtain a main scan magnetic resonance (MR) image (referred to as a diagnostic image). MR data from pre-scans can be used, for example, to determine sensitivity maps for RF coils 975 and/or 979 (sometimes referred to as coil sensitivity maps or spatial sensitivity maps), and to determine unfolding maps for parallel imaging.

The MRI system components 972 include an RF receiver 989 providing input to data processor 990 so as to create processed image data, which is sent to display 980. The MRI data processor 990 is also configured to access previously generated MR data, images, and/or maps, such as, for example, coil sensitivity maps, parallel image unfolding maps, distortion maps and/or system configuration parameters 991, and MRI image reconstruction program code structures 992 and 993.

In one embodiment, the MRI data processor 990 includes processing circuitry. The processing circuitry can include devices such as an application-specific integrated circuit (ASIC), configurable logic devices (e.g., simple programmable logic devices (SPLDs), complex programmable logic devices (CPLDs), and field programmable gate arrays (FPGAs), and other circuit components that are arranged to perform the functions recited in the present disclosure.

The processor 990 executes one or more sequences of one or more instructions, such as method 100 described herein, contained in the program code structures 992 and 993. Alternatively, the instructions can be read from another computer-readable medium, such as a hard disk or a removable media drive. One or more processors in a multi-processing arrangement can also be employed to execute the sequences of instructions contained in the program code structures 992 and 993. In alternative embodiments, hard-wired circuitry can be used in place of or in combination with software instructions. Thus, the disclosed embodiments are not limited to any specific combination of hardware circuitry and software.

Additionally, the term "computer-readable medium" as used herein refers to any non-transitory medium that participates in providing instructions to the processor 990 for execution. A computer-readable medium can take many forms, including, but not limited to, non-volatile media or volatile media. Non-volatile media includes, for example, optical, magnetic disks, and magneto-optical disks, or a removable media drive. Volatile media includes dynamic memory.

Also illustrated in FIG. 9, and as referenced above, is a generalized depiction of an MRI system program storage (memory) 993, where stored program code structures are stored in non-transitory computer-readable storage media accessible to the various data processing components of the MRI system 970. As those in the art will appreciate, the program store 993 can be segmented and directly connected, at least in part, to different ones of the system 972 processing computers having most immediate need for such stored program code structures in their normal operation (i.e., rather than being commonly stored and connected directly to the MRI system controller 983).

Additionally, the MRI system 970 as depicted in FIG. 9 can be utilized to practice exemplary embodiments described herein below. The system components can be divided into different logical collections of "boxes" and typically comprise numerous digital signal processors (DSP), microprocessors and special purpose processing circuits (e.g., for fast A/D conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Furthermore, not only does the physical state of the processing circuits (e.g., CPUs, registers, buffers, arithmetic units, etc.) progressively change from one clock cycle to another during the course of operation, the physical state of associated data storage media (e.g., bit storage sites in magnetic storage media) is transformed from one state to another during operation of such a system. For example, at the conclusion of an image reconstruction process and/or sometimes an image reconstruction map (e.g., coil sensitivity map, unfolding map, ghosting map, a distortion map etc.) generation process, an array of computer-readable accessible data value storage sites in physical storage media will be transformed from some prior state (e.g., all uniform "zero" values or all "one" values) to a new state wherein the physical states at the physical sites of such an array vary between minimum and maximum values to represent real world physical events and conditions (e.g., the internal physical structures of a patient over an imaging volume space). As those in the art will appreciate, such arrays of stored data values represent and also constitute a physical structure, as does a particular structure of computer control program codes that, when sequentially loaded into instruction registers and executed by one or more CPUs of the MRI system 970, causes a particular sequence of operational states to occur and be transitioned through within the MRI system 970.

Numerous modifications and variations of the present disclosure are possible in light of the above teachings provided that such modifications and variations fall within the scope of the appended claims.

**Claims**

1. An apparatus configured to reconstruct or filter medical image data, the apparatus comprising:
   processing circuitry configured to

   receive a first medical image data and meta-parameters related to acquisition of the first medical image data;
   apply the received meta-parameters to inputs of a first trained machine-learning 'ML' network to obtain, from outputs of the first trained ML network, tuning parameters of a second ML network different from the first ML network;
   apply the received first medical image data to inputs of the second ML network, as tuned by the obtained tuning parameters output from the first ML network, to obtain, from outputs of the second ML network, second medical image data; wherein the second ML network is configured to perform inference of one of a reconstruction function and a filtering function based on the input first medical image data; and
   output the second medical image data, wherein:

   the second ML network is unrolled for a fixed number of iterations, alternating between a data consistency 'DC' enforcing step and a convolutional neural network 'CNN';
   the tuning parameters include a regularization parameter in the DC enforcing step and feature scaling vectors that scale output vectors of corresponding intermediate layers of the CNN,
   wherein the first ML network and the second ML network have been jointly trained using training data and a single loss function.

2. The apparatus of claim 1, wherein the first medical image data received by the processing circuitry is magnetic-resonance k-space data and the second medical data output by the second ML network is a magnetic-resonance image.

3. The apparatus of claim 1, wherein the processing circuitry is further configured to apply the received meta-parameters to the first ML network, which is a multilayer perceptron 'MLP' network.

4. The apparatus of claim 1, wherein the first image data is one of magnetic resonance imaging 'MRI' data, computed tomography 'CT' data, and positron emission tomography 'PET' data.

5. The apparatus of claim 1, wherein the first medical image data is magnetic-resonance image data and the meta-parameters include a T1 parameter, a T2 parameter, a fluid-attenuated inversion recovery 'FLAIR' parameter, a field strength parameter, and an acceleration parameter.

6. The apparatus of claim 1, wherein the second ML network is configured to perform inference of one of a reconstruction function and a filtering function based on the input first medical image data.

7. A method for reconstructing or filtering medical image data, the method comprising:

receiving a first medical image data and meta-parameters related to acquisition of the first medical image data;
applying the received meta-parameters to inputs of a first trained machine-learning (ML) network to obtain, from outputs of the first trained ML network, tuning parameters of a second ML network different from the first ML network;
applying the received first medical image data to inputs of the second ML network, as tuned by the obtained tuning parameters output from the first ML network, to obtain, from outputs of the second ML network, second medical image data; wherein the second ML network is configured to perform inference of one of a reconstruction function and a filtering function based on the input first medical image data; and
outputting the second medical image data, wherein:

the second ML network is unrolled for a fixed number of iterations, alternating between a data consistency 'DC' enforcing step and a convolutional neural network 'CNN';
the tuning parameters include a regularization parameter in the DC enforcing step and feature scaling vectors that scale output vectors of corresponding intermediate layers of the CNN,
wherein the first ML network and the second ML network have been jointly trained using training data and a single loss function.

8. The method of claim 7, wherein the first medical image data received in the receiving step is magnetic-resonance k-space data and the second medical data output by the second ML network is a magnetic-resonance image.

9. The method of claim 7, wherein the step of applying the received meta-parameters to the first ML network comprises applying the received meta-parameters a multilayer perceptron 'MLP' network.

10. The method of claim 7, wherein the first image data is one of magnetic resonance imaging 'MRI' data, computed tomography 'CT' data, and positron emission tomography 'PET' data.

11. The method of claim 7, wherein the first medical image data is magnetic-resonance image data and the meta-parameters include a T1 parameter, a T2 parameter, a fluid-attenuated inversion recovery 'FLAIR' parameter, a field strength parameter, and an acceleration parameter.

12. A computer-readable medium storing a program that, when executed by processing circuitry, causes the processing circuitry to execute a method for reconstructing or filtering medical image data according to one of claims 7 to 11.

**Patentansprüche**

1. Einrichtung, die dafür konfiguriert ist, medizinische Bilddaten zu rekonstruieren oder zu filtern, wobei die Einrichtung Folgendes umfasst:

Verarbeitungsschaltungen, die zu Folgendem konfiguriert sind:

erste medizinische Bilddaten und Metaparameter, die mit der Erfassung der ersten medizinischen Bilddaten in Beziehung stehen, zu empfangen;

die empfangenen Metaparameter auf Eingaben eines ersten trainierten Netzwerks zum Maschinellen Lernen, ML, anzuwenden, um, aus Ausgaben des ersten trainierten ML-Netzwerks, Abstimmungsparameter eines zweiten ML-Netzwerks, das sich vom ersten ML-Netzwerk unterscheidet, zu erhalten;

die empfangenen ersten medizinischen Bilddaten auf Eingaben des zweiten ML-Netzwerks anzuwenden, wie durch die erhaltenen vom ersten ML-Netzwerk ausgegebenen Abstimmungsparameter abgestimmt, um, aus Ausgaben des zweiten ML-Netzwerks, zweite medizinische Bilddaten zu erhalten; wobei das zweite ML-Netzwerk dafür konfiguriert ist, auf Grundlage der eingegebenen ersten medizinischen Bilddaten eine Schlussfolgerung auf eines von einer Rekonstruktionsfunktion und einer Filterfunktion durchzuführen; und

die zweiten medizinischen Bilddaten auszugeben, wobei:

das zweite ML-Netzwerk für eine feste Anzahl von Iterationen abgewickelt wird, wobei zwischen einem Schritt zur Durchsetzung der Datenkonsistenz (DC) und einem Neuronalen Faltungsnetz (CNN) abgewechselt wird;

die Abstimmungsparameter einen Regularisierungsparameter im DC-Durchsetzungsschritt und Merkmalsskalierungsvektoren, die Ausgabevektoren entsprechender Zwischenschichten des CNN skalieren, einschließen,

wobei das erste ML-Netzwerk und das zweite ML-Netzwerk gemeinsam unter Verwendung von Trainingsdaten und einer einzigen Verlustfunktion trainiert worden sind.

2. Einrichtung nach Anspruch 1, wobei die ersten von der Verarbeitungsschaltungen empfangenen medizinischen Bilddaten Magnetresonanz-k-Raum-Daten sind und die zweiten von dem zweiten ML-Netzwerk ausgegebenen medizinischen Daten ein Magnetresonanzbild sind.

3. Einrichtung nach Anspruch 1, wobei die Verarbeitungsschaltungen ferner dafür konfiguriert sind, die empfangenen Metaparameter auf das erste ML-Netzwerk anzuwenden, das ein Mehrlagen-Perzeptron- (MLP-)Netzwerk ist.

4. Einrichtung nach Anspruch 1, wobei die ersten Bilddaten eines von Magnetresonanztomographie-, MRT-, Daten, Computertomographie- (CT-)Daten und Positronenemissionstomographie- (PET-)Daten sind.

5. Einrichtung nach Anspruch 1, wobei die ersten medizinischen Bilddaten Magnetresonanzbilddaten sind und die Metaparameter einen T1-Parameter, einen T2-Parameter, einen Fluid-Attenuated-Inversion-Recovery- (FLAIR-) Parameter, einen Feldstärkeparameter und einen Beschleunigungsparameter einschließen.

6. Einrichtung nach Anspruch 1, wobei das zweite ML-Netzwerk dafür konfiguriert ist, auf Grundlage der eingegebenen ersten medizinischen Bilddaten eine Schlussfolgerung auf eines von einer Rekonstruktionsfunktion und einer Filterfunktion durchzuführen.

7. Verfahren zum Rekonstruieren oder Filtern medizinischer Bilddaten, wobei das Verfahren Folgendes umfasst:

Empfangen von ersten medizinischen Bilddaten und Metaparametern, die mit der Erfassung der ersten medizinischen Bilddaten in Beziehung stehen;

Anwenden der empfangenen Metaparameter auf Eingaben eines ersten trainierten Netzwerks zum Maschinellen Lernen (ML) um, aus Ausgaben des ersten trainierten ML-Netzwerks, Abstimmungsparameter eines zweiten ML-Netzwerks, das sich vom ersten ML-Netzwerk unterscheidet, zu erhalten;

Anwenden der empfangenen ersten medizinischen Bilddaten auf Eingaben des zweiten ML-Netzwerks, wie durch die erhaltenen vom ersten ML-Netzwerk ausgegebenen Abstimmungsparameter abgestimmt, um, aus Ausgaben des zweiten ML-Netzwerks, zweite medizinische Bilddaten zu erhalten; wobei das zweite ML-Netzwerk dafür konfiguriert ist, auf Grundlage der eingegebenen ersten medizinischen Bilddaten eine Schlussfolgerung auf eines von einer Rekonstruktionsfunktion und einer Filterfunktion durchzuführen; und

Ausgeben der zweiten medizinischen Bilddaten, wobei:

das zweite ML-Netzwerk für eine feste Anzahl von Iterationen abgewickelt wird, wobei zwischen einem Schritt zur Durchsetzung der Datenkonsistenz (DC) und einem Neuronalen Faltungsnetz (CNN) abgewechselt wird;

die Abstimmungsparameter einen Regularisierungsparameter im DC-Vektoren-Durchsetzungsschritt und Merkmalsskalierungsvektoren, die Ausgabevektoren entsprechender Zwischenschichten des CNN skalieren, einschließen,

wobei das erste ML-Netzwerk und das zweite ML-Netzwerk gemeinsam unter Verwendung von Trainingsdaten und einer einzigen Verlustfunktion trainiert worden sind.

**8.** Verfahren nach Anspruch 7, wobei die ersten im Empfangsschritt empfangenen medizinischen Bilddaten Magnetresonanz-k-Raum-Daten sind und die zweiten von dem zweiten ML-Netzwerk ausgegebenen medizinischen Daten ein Magnetresonanzbild sind.

**9.** Verfahren nach Anspruch 7, wobei der Schritt des Anwendens der empfangenen Metaparameter auf das erste ML-Netzwerk das Anwenden der empfangenen Metaparameter auf ein Mehrlagen-Perzeptron- (MLP-)Netzwerk umfasst.

**10.** Verfahren nach Anspruch 7, wobei die ersten Bilddaten eines von Magnetresonanztomographie-, MRT-, Daten, Computertomographie- (CT-)Daten und Positronenemissionstomographie- (PET-)Daten sind.

**11.** Verfahren nach Anspruch 7, wobei die ersten medizinischen Bilddaten Magnetresonanzbilddaten sind und die Metaparameter einen T1-Parameter, einen T2-Parameter, einen Fluid-Attenuated-Inversion-Recovery-(FLAIR-) Parameter, einen Feldstärkeparameter und einen Beschleunigungsparameter einschließen.

**12.** Computerlesbares Medium, das ein Programm speichert, das, wenn es von Verarbeitungsschaltungen ausgeführt wird, die Verarbeitungsschaltungen veranlasst, ein Verfahren zum Rekonstruieren oder Filtern medizinischer Bilddaten nach einem der Ansprüche 7 bis 11 auszuführen.


## Revendications

**1.** Appareil configuré pour reconstruire ou filtrer des données d'imagerie médicale, l'appareil comprenant :

un circuit de traitement configuré pour

recevoir de premières données d'imagerie médicale et des méta-paramètres liés à l'acquisition de ces premières données d'imagerie médicale ;
appliquer les métaparamètres reçus aux entrées d'un premier réseau d'apprentissage automatique « ML » entraîné afin d'obtenir, à partir des sorties du premier réseau ML entraîné, des paramètres de réglage d'un deuxième réseau ML différent du premier réseau ML;
appliquer les premières données d'imagerie médicale reçues aux entrées du deuxième réseau ML, tel que réglé par les paramètres de réglage obtenus à partir du premier réseau ML, afin d'obtenir, à partir des sorties du deuxième réseau ML, des deuxièmes données d'imagerie médicale ; dans lequel le deuxième réseau ML est configuré pour effectuer l'inférence d'une fonction de reconstruction ou d'une fonction de filtrage à partir des premières données d'imagerie médicale en entrée ; et
produire les deuxièmes données d'imagerie médicale, dans lequel :

le deuxième réseau ML est déroulé pendant un nombre fixe d'itérations, en alternant entre une étape d'application de la cohérence des données « DC » et un réseau neuronal convolutif « CNN » ;
les paramètres de réglage incluent un paramètre de régularisation dans l'étape d'application du DC et des vecteurs de mise à l'échelle des caractéristiques qui mettent à l'échelle les vecteurs de sortie des couches intermédiaires correspondantes du CNN.

dans lequel le premier réseau ML et le deuxième réseau ML ont été entraînés conjointement à l'aide de données d'entraînement et d'une fonction de perte unique

**2.** Appareil selon la revendication 1, dans lequel les premières données d'imagerie médicale reçues par le circuit de traitement sont des données d'espace k de résonance magnétique et les deuxièmes données médicales produites par le deuxième réseau ML sont une image de résonance magnétique.

**3.** Appareil selon la revendication 1, dans lequel le circuit de traitement est en outre configuré pour appliquer les métaparamètres reçus au premier réseau ML, qui est un réseau de perceptron multicouche « MLP ».

4. Appareil selon la revendication 1, dans lequel les premières données d'imagerie sont des données d'imagerie par résonance magnétique « IRM », des données de tomodensitométrie « CT » et des données de tomographie par émission de positons « PET ».

5. Appareil selon la revendication 1, dans lequel les premières données d'imagerie médicale sont des données d'imagerie par résonance magnétique et les métaparamètres incluent un paramètre T1, un paramètre T2, un paramètre de récupération d'inversion atténuée par le fluide « FLAIR », un paramètre de force de champ et un paramètre d'accélération.

6. Appareil selon la revendication 1, dans lequel le deuxième réseau ML est configuré pour effectuer l'inférence de l'une des fonctions de reconstruction et d'une fonction de filtrage à partir des premières données d'imagerie médicale d'entrée.

7. Procédé de reconstruction ou de filtrage de données d'imagerie médicales, le procédé comprenant :

la réception des premières données d'imagerie médicale et des métaparamètres liés à l'acquisition de ces premières données d'imagerie médicale ;

l'application des métaparamètres reçus aux entrées d'un premier réseau d'apprentissage automatique (ML) entraîné pour obtenir, à partir des sorties du premier réseau ML entraîné, les paramètres de réglage d'un deuxième réseau ML différent du premier réseau ML;

l'application des premières données d'imagerie médicale reçues aux entrées du deuxième réseau d'apprentissage automatique, tel que réglé par les paramètres de réglage obtenus à partir du premier réseau d'apprentissage automatique, afin d'obtenir, à partir des sorties du deuxième réseau d'apprentissage automatique, des deuxièmes données d'imagerie médicale ; dans lequel le deuxième réseau d'apprentissage automatique est configuré pour effectuer l'inférence d'une fonction de reconstruction ou d'une fonction de filtrage à partir des premières données d'imagerie médicale en entrée ; et

la production des deuxièmes données d'imagerie médicale, dans lequel :

le deuxième réseau ML est déroulé pendant un nombre fixe d'itérations, en alternant entre une étape d'application de la cohérence des données « DC » et un réseau neuronal convolutif « CNN » ;

les paramètres de réglage incluent un paramètre de régularisation dans les vecteurs DC imposant un pas et des vecteurs de mise à l'échelle des caractéristiques qui mettent à l'échelle les vecteurs de sortie des couches intermédiaires correspondantes du CNN.

dans lequel le premier réseau ML et le deuxième réseau ML ont été entraînés conjointement à l'aide de données d'entraînement et d'une fonction de perte unique,

8. Procédé selon la revendication 7, dans lequel les premières données d'imagerie médicale reçues à l'étape de réception sont des données d'espace k de résonance magnétique et les deuxièmes données médicales produites par le deuxième réseau ML sont une image de résonance magnétique.

9. Procédé selon la revendication 7, dans lequel l'étape d'application des métaparamètres reçus au premier réseau ML comprend l'application des métaparamètres reçus à un réseau de perceptron multicouche « MLP ».

10. Procédé selon la revendication 7, dans lequel les premières données d'imagerie sont des données d'imagerie par résonance magnétique « IRM », des données de tomodensitométrie « TDM » et des données de tomographie par émission de positons « TEP ».

11. Procédé selon la revendication 7, dans lequel les premières données d'imagerie médicale sont des données d'imagerie par résonance magnétique et les métaparamètres incluent un paramètre T1, un paramètre T2, un paramètre « FLAIR » (Fluid-Attenuated Inversion Recovery), un paramètre de force de champ et un paramètre d'accélération.

12. Support lisible par ordinateur stockant un programme qui, lorsqu'il est exécuté par un circuit de traitement, amène le circuit de traitement à exécuter un procédé de reconstruction ou de filtrage de données d'imagerie médicales selon l'une des revendications 7 à 11.

FIG. 1A

FIG. 1B

FIG. 1C

EP 4 365 618 B1

FIG. 2

FIG. 3

FIG. 4

EP 4 365 618 B1

FIG. 5

reference (1x)    MoDL-I (4x)    MoDL (4x)    Meta-MoDL (4x)
(individual)    (universal)

FIG. 6A

FIG. 6B

reference (1x)          MoDL (4x)          Meta-MoDL (4x)
                        (universal)

FIG. 6C

EP 4 365 618 B1

Meta-MoDL (4x)  MoDL (4x) (universal)  reference (1x)

FIG. 6D

PSNR (dB), 4x

SSIM (x 10³), 4x

FIG. 7

EP 4 365 618 B1

*FIG. 8*

EP 4 365 618 B1

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 63422594 A

**Non-patent literature cited in the description**

- **ZHANG HUIXIAN et al.** Multi-Contrast MRI Image Synthesis Using Switchable Cycle-Consistent Generative Adversarial Networks. *Diagnostics*, 20 March 2022, vol. 12 (4), 816
- Continuous Conversion of CT Kernel Using Switchable CycleGAN With AdaIN. **YANG SERIN et al.** IEEE TRANSACTIONS ON MEDICAL IMAGING. IEEE, 05 May 2021, vol. 40, 3015-3029
- MoDL: Model Based Deep Learning Architecture for Inverse Problems. **HEMANT KUMAR AGGARWAL et al.** ARXIV.ORG. CORNELL UNIVERSITY LIBRARY, 07 December 2017